(19) Europäisches Patentamt
European Patent Office
Office européen des brevets

(11) **EP 4 314 808 B1**

(12) **FASCICULE DE BREVET EUROPEEN**

(45) Date de publication et mention
de la délivrance du brevet:
**22.01.2025 Bulletin 2025/04**

(21) Numéro de dépôt: **22714190.0**

(22) Date de dépôt: **14.03.2022**

(51) Classification Internationale des Brevets (IPC):
**G01N 33/24** *(2006.01)*    **G01N 31/12** *(2006.01)*

(52) Classification Coopérative des Brevets (CPC):
**G01N 33/24; G01N 31/12**

(86) Numéro de dépôt international:
**PCT/EP2022/056458**

(87) Numéro de publication internationale:
**WO 2022/200093 (29.09.2022 Gazette 2022/39)**

(54) **PROCEDE POUR LA QUANTIFICATION ET LA CARACTERISATION DU CARBONE DANS LES SOLS**

VERFAHREN ZUR QUANTIFIZIERUNG UND CHARAKTERISIERUNG VON KOHLENSTOFF IN BÖDEN

METHOD FOR THE QUANTIFICATION AND CHARACTERIZATION OF CARBON IN SOILS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.03.2021 FR 2103114**

(43) Date de publication de la demande:
**07.02.2024 Bulletin 2024/06**

(73) Titulaire: **IFP Energies nouvelles**
**92500 Rueil-Malmaison (FR)**

(72) Inventeurs:
• **SEBAG, David**
  **92852 RUEIL-MALMAISON CEDEX (FR)**
• **KOWALEWSKI, Isabelle**
  **92852 RUEIL-MALMAISON CEDEX (FR)**
• **LAMOUREUX-VAR, Violaine**
  **92852 RUEIL-MALMAISON CEDEX (FR)**
• **PILLOT, Daniel**
  **92852 RUEIL-MALMAISON CEDEX (FR)**
• **RAVELOJAONA, Herman**
  **92852 RUEIL-MALMAISON CEDEX (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
• **F. BEHAR ET AL: "Rock-Eval 6 Technology: Performances and Developments", OIL & GAS SCIENCE AND TECHNOLOGY &NDASH; REVUE DE L&RSQUO;INSTITUT FRANÇAIS DU PÉTROLE, vol. 56, no. 2, 1 March 2001 (2001-03-01), pages 111 - 134, XP055113461, ISSN: 1294-4475, DOI: 10.2516/ogst:2001013**
• **JOHANNES ET AL: "Evaluation of oil potential and pyrolysis kinetics of renewable fuel and shale samples by Rock-Eval analyzer", JOURNAL OF ANALYTICAL AND APPLIED PYROLYSIS, ELSEVIER BV, NL, vol. 79, no. 1-2, 19 April 2007 (2007-04-19), pages 183 - 190, XP022062360, ISSN: 0165-2370, DOI: 10.1016/ J.JAAP.2006.12.001**
• **MALOU OSCAR PASCAL ET AL: "The Rock-Eval signature of soil organic carbon in arenosols of the Senegalese groundnut basin. How do agricultural practices matter?", AGRICULTURE, ECOSYSTEMS AND ENVIRONMENT, ELSEVIER, AMSTERDAM, NL, vol. 301, 26 June 2020 (2020-06-26), XP086227123, ISSN: 0167-8809, [retrieved on 20200626], DOI: 10.1016/ J.AGEE.2020.107030**

## Description

### Domaine technique

La présente invention concerne le domaine de la science du sol et des géosciences de l'environnement. Plus particulièrement la caractérisation du carbone contenu dans les formations superficielles, et notamment dans les sols.

Afin de répondre aux enjeux écologiques, ou de respecter certaines législations/directives environnementales, les acteurs de la science du sol et des géosciences de l'environnement (laboratoires de recherche, bureaux d'étude, agences environnementales, exploitants agricoles) sont de plus en plus souvent amenés à mettre en place des protocoles de suivi des impacts des activités humaines sur les stocks de carbone des sols et des éco-agro-systèmes. Ces suivis et ces études d'impact impliquent de pouvoir étudier de larges séries d'échantillons dans des délais relativement courts au regard des méthodologies classiquement employées. De plus, ces méthodes s'accompagnent souvent de contraintes environnementales et sécuritaires qui augmentent les délais et les coûts analytiques, et nécessitent souvent de faire appel à des prestataires spécialisés (par exemple des laboratoires d'analyse).

Les formes organiques du carbone stockées dans les formations superficielles, et notamment dans les sols, représentent un enjeu majeur pour l'agriculture et le climat. Elles jouent en effet un rôle primordial sur la qualité structurelle et la valeur fertilisante des sols, mais interviennent surtout dans le cycle du carbone, en représentant le plus important réservoir de carbone organique à la surface de la Terre. Assurer la sécurité alimentaire des populations et favoriser la séquestration du carbone organique dans les sols nécessite de trouver un compromis entre maintenir des stocks de matière organique suffisamment labiles pour libérer les nutriments nécessaires à la croissance végétale tout en favorisant le stockage de formes de carbone suffisamment résistantes pour atténuer, à long terme, les émissions anthropogéniques de gaz à effet de serre.

Les formes inorganiques de carbone sont de minéralogie variable. Essentiellement représentées par des carbonates et des oxalates de calcium (Ca) dans les sols, elles peuvent parfois intégrer d'autres cations (Fe dans la sidérite, Mg dans la dolomite, etc.), notamment en domaine tropical. Indépendamment de leur rôle dans les cycles biogéochimiques (notamment comme puits de carbone), les formes minérales du carbone posent des problèmes techniques pour l'analyse des formes organiques. En effet, aucune méthode de routine ne permet de caractériser les différentes formes de carbone à l'aide d'une seule et unique mesure, et les approches classiquement employées reposent sur des extractions et des séparations spécifiques (fumigation acide, méthode Walkley and Black, calcimètrie Bernard) suivies d'analyses ciblées à l'aide des équipements scientifiques spécialisés (analyse élémentaire CHN, spectrométrie IR, GC-MS, $^{13}$C-RMN).

On connait aussi des méthodes d'analyse thermique de la matière organique des sols reposant sur des mesures de quantités de composés hydrocarbonés (HC), de monoxyde de carbone (CO) et/ou de dioxyde de carbone ($CO_2$) libérées au cours du temps par un échantillon soumis à une séquence de chauffe en atmosphère inerte puis/ou à une séquence de chauffe en atmosphère oxydante. Ces méthodes ont été développées initialement dans le domaine de l'industrie pétrolière, à des fins de caractérisation de la fraction organique des roches sédimentaires. On connaît ainsi le dispositif ROCK-EVAL® (IFP Energies nouvelles, France), développé par la demanderesse et décrit notamment dans les brevets FR 2227797 (US 3953171) et FR 2472754 (US 4352673), qui comprend un four de pyrolyse distinct d'un four d'oxydation, un détecteur du type à ionisation de flamme (FID) pour détecter les composés hydrocarbonés (HC) et un détecteur du type infrarouge (IR) pour détecter le monoxyde de carbone (CO) et/ou le dioxyde de carbone ($CO_2$). On connait en outre des procédés développés pour des applications particulières du domaine de l'industrie pétrolière, ayant chacun leur propre séquence de températures de chauffe en pyrolyse et/ou de chauffe en atmosphère oxydante. Notamment, on connait la méthode "ROCK-EVAL® BULK ROCK", dédiée plus particulièrement aux échantillons conventionnels de roches mères (Behar et al., 2001). La séquence de chauffe en atmosphère inerte de cette méthode est caractérisée par une température initiale T1 du four de pyrolyse généralement comprise entre 300°C et 350°C, température qui est maintenue pendant une durée prédéterminée de quelques minutes. C'est durant cette phase que sont libérés les hydrocarbures dits « libres » (correspondant en réalité à des hydrocarbures de poids moléculaire léger à lourd) initialement contenus dans l'échantillon de roche. Leur quantité est estimée via la mesure de la surface d'un premier pic, noté $S_1$, de la courbe (aussi appelée thermogramme) représentant la quantité de composés hydrocarbonés libérés pendant la séquence de chauffe en atmosphère inerte. Puis, la température de pyrolyse est augmentée progressivement jusqu'à une température T2, généralement de 650°C. Durant cette phase, on assiste à la volatilisation des composés hydrocarbonés très lourds, ainsi qu'au craquage de la matière organique non volatile (kérogène). La quantité de composés hydrocarbonés libérés durant cette phase de craquage thermique est estimée via la mesure de la surface d'un second pic, noté S2. En parallèle, les quantités de CO et de $CO_2$ sont mesurées et représentées également sous la forme de courbes. Ces courbes présentent deux pics classiquement notés S3CO (respectivement S3CO2), qui est considéré comme correspondant au CO (respectivement $CO_2$) généré par le craquage de la matière organique de l'échantillon pendant la chauffe sous atmosphère inerte, et S3'CO (respectivement S3'CO2) qui est considéré comme correspondant au CO (respectivement $CO_2$) généré par la décomposition thermique des formes carbonatées (notamment la calcite) pendant la chauffe sous atmosphère inerte. Puis le résidu de l'échantillon issu de la chauffe en atmosphère inerte est soumis à une chauffe en atmosphère oxydante : à partir d'une température comprise entre environ 300°C et 400°C, et valant de préférence 300°C, on élève la température du résidu de l'échantillon considéré selon un gradient de température compris entre 20 et 40°C/minute, jusqu'à une température de fin d'oxydation comprise entre 750 et 950°C, et valant de préférence 850°C. Durant cette séquence de chauffe en atmosphère oxydante, les quantités de CO et de $CO_2$ libérées par le résidu de l'échantillon sont mesurées et représentées sous la forme de courbes, conduisant à un pic classiquement noté S4CO (respectivement S4CO2) qui est considéré comme correspondant à la quantité de CO (respectivement $CO_2$) généré par la combustion de la matière organique pendant le cycle d'oxydation. A partir de ces mesures, cette méthode définit un certain nombre de paramètres standards, en particulier le paramètre noté TOC (pour "Total Organic Carbon" en anglais) qui correspond à la teneur en carbone de l'échantillon, déterminée à partir de la quantité totale de HC libérée par l'échantillon et des quantités de CO et de $CO_2$ libérées en-dessous de températures-seuils pendant la phase de pyrolyse et la phase d'oxydation ; et le paramètre noté MinC (pour "Mineral Carbon" en anglais) qui correspond à la teneur en carbone minéral de l'échantillon, déterminée à partir des quantités de CO et de $CO_2$ libérées par l'échantillon au-dessus de températures-seuils pendant la phase de pyrolyse et la phase d'oxydation.

**Technique antérieure**

Les documents suivants seront cités au cours de la description :

Behar F., Beaumont V., De B., Penteado H.L. (2001) Rock-Eval 6 Technology: Performances and Developments, Oil & Gas Science and Technology 56, 111-134.

Disnar, J.R., Guillet, B., Keravis, D., Di-Giovanni, C., Sebag, D., 2003. Soil organic matter (SOM) characterization by Rock-Eval pyrolysis: scope and limitations. Organic Geochemistry 34, 327-343.

Malou, O.P., Sebag, D., Moulin P., Chevallier, T., Badiane-Ndour, N.Y., Thiam, A., Chapuis-Lardy, L. 2020. The Rock-Eval® signature of soil organic carbon in Arenosols of the Senegalese groundnut Basin. How do agricultural practices matter? » Agriculture, Ecosystems & Environment 301: 107030. https://doi.org/10.1016/j.agee.2020.107030.

Pillot, D., Deville, E., Prinzhofer, A., 2014. Identification and Quantification of Carbonate Species Using Rock-Eval Pyrolysis. Oil & Gas Science and Technology - Revue d'IFP Energies nouvelles 69, 341-349.

Sebag, D., Disnar, J.R., Guillet, B., Di Giovanni, C., Verrecchia, E.P., Durand, A., 2006. Monitoring organic matter dynamics in soil profiles by "Rock-Eval pyrolysis": bulk characterization and quantification of degradation. European Journal of Soil Science 57, 344-355.

Sebag, D., Verrecchia, E.P., Cécillon, L., Adatte, T., Albrecht, R., Aubert, M., Bureau, F., Cailleau, G., Copard, Y., Decaëns, T., Disnar, J.-R., Hetényi, M., Nyilas, T., Trombino, L., 2016. Dynamics of soil organic matter based on new Rock-Eval indices. Geoderma 284, 185-203.

Depuis les années 2000, ce type d'analyses a également été utilisé et adapté pour étudier la fraction organique des formations superficielles et notamment des sols.

Par exemple on connait la méthode décrite dans le document (Disnar et al., 2003), dans lequel est proposée une adaptation de la séquence de chauffe en pyrolyse consistant en une température initiale de la séquence de chauffe en atmosphère inerte valant 200°C, au lieu de 300°C dans la méthode "ROCK-EVAL® BULK ROCK" décrite précédemment. Cette température initiale plus basse permet de d'étendre le pic S2 défini précédemment aux températures de craquage des composants organiques les plus thermolabiles. Ces constituants thermolabiles sont en effet beaucoup plus abondants dans les formations superficielles que dans les roches sédimentaires. Pour estimer leur contribution et ainsi évaluer la stabilité thermique de la matière organique, cette méthode définit le paramètre R400 qui mesure la proportion relative du pic S2 correspondant aux températures inférieures à 400°C. Par ailleurs, ce même document met en évidence un écart négatif entre le paramètre TOC tel que défini dans la méthode ROCK-EVAL® BULK ROCK et les teneurs en carbone organique mesurées avec des méthodes normalisées (par exemple des analyses élémentaires) et recommande une correction statistique (c'est-à-dire l'application d'un coefficient correcteur établi sur un panel représentatif d'échantillons de sols) pour corriger le paramètre TOC, défini pour le domaine pétrolier, afin qu'il soit vraiment représentatif de la teneur en carbone organique, notamment pour les échantillons riches en matières organiques peu décomposées par rapport à leurs précurseurs biogéniques (litières, composts, tourbes, etc.).

On connait également le document (Sebag et al., 2006) qui utilise cette séquence de chauffe adaptée, et qui propose en complément une déconvolution du pic S2 pour évaluer le degré de décomposition des constituants organiques. En effet, les thermogrammes des formations superficielles, en particulier des échantillons organiques, présentent une distribution plurimodale dont les modes principaux sont toujours situés dans des intervalles de température particuliers (300-320°C ; 360-380°C ; 420-440°C ; 470-490°C et 540-560°C). La méthode décrite dans ce document consiste à décomposer le pic S2 en cinq distributions gaussiennes élémentaires centrées sur ces modes. Cette déconvolution mathématique revient à estimer la contribution relative au pic S2 que chaque distribution élémentaire considérée comme relative à une classe de constituants définis par leur seule température de craquage. Ces contributions élémentaires sont ensuite utilisées pour calculer de nouveaux paramètres qui mesurent la stabilité thermique globale de la matière organique (R-index) et le degré de décomposition de la fraction thermolabile (I-index). Cependant, la méthode de déconvolution repose sur une approche itérative pour ajuster la position et la surface de chaque distribution élémentaire en fonction de paramètres statistiques fixés arbitrairement, ce qui réduit la reproductibilité de la décomposition.

On connait également le document (Sebag et al., 2016) qui décrit une approche alternative en montrant qu'une intégration des thermogrammes représentatifs de la quantité de HC contenus dans l'échantillon par bandes de température (200-340°C ; 340-400°C ; 400-460°C ; 460-520°C et 520-650°C) aboutit à des résultats comparables à la déconvolution mathématique, et sont, qui plus est, parfaitement reproductibles. Cette approche a été utilisée pour de nombreuses applications, mais souffre de quelques limites intrinsèques. Tout d'abord, elle repose sur la définition de températures-seuils définies empiriquement pour que la surface de chaque bande de température approche au mieux la surface de la distribution élémentaire correspondante obtenue par déconvolution. En outre, ces surfaces et les paramètres qui en découlent ne fournissent qu'une information qualitative dans la mesure où les unes comme les autres correspondent à des proportions relatives ou des rapports de proportions relatives. Enfin, cette approche se limite à l'examen des quantités de HC émises au cours de la phase de pyrolyse, alors que la majorité du carbone est représentée par le CO et le $CO_2$ émis au cours des phases de pyrolyse et d'oxydation.

En outre, malgré ces tentatives d'adaptation ou d'amélioration, les méthodes connues ne fournissent pas des valeurs des paramètres standards TOC et MINC qui soient représentatives respectivement de la teneur d'un échantillon en carbone organique et en carbone minéral dans le cas de formations superficielles. En effet, le paramètre TOC (respectivement MINC) de la méthode "ROCK-EVAL® BULK ROCK" mesure la teneur en carbone à partir des quantités de CO et de $CO_2$ émises en-dessous (respectivement au-dessus) de températures-seuils considérées comme des limites thermiques entre les formes organiques et minérales de carbone.

Or cette définition est erronée que ce soit pour des formations carbonatées ou non carbonatées. En effet, comme montré dans le document (Malou et al. 2020) cette définition implique notamment le calcul systématique d'un paramètre MINC, même pour les échantillons sans forme minérale de carbone. Mais plus généralement, comme constaté par la demanderesse, ceci implique que, même dans les échantillons contenant des formes minérales de carbone, une part du MINC provient du craquage thermique des constituants organiques au-dessus des températures-seuils. Enfin, comme montré dans le document (Pillot et al. 2014), certaines formes minérales de carbone (comme la sidérite ou les oxalates) sont susceptibles de se décomposer à des températures inférieures aux températures-seuils utilisées pour les calculs des paramètres TOC et de MINC standards.

La présente invention vise à pallier les inconvénients liés à l'utilisation d'un procédé initialement développé pour le domaine pétrolier pour analyser la matière organique des formations superficielles, et notamment des sols. En particulier, la présente invention définit une séquence de chauffe sous atmosphère inerte modifiée, sous la forme d'un gradient thermique et donc restant simple de mise en oeuvre, mais mieux adaptée à des échantillons d'une formation superficielle. Une séquence de chauffe sous atmosphère oxydante optionnelle, également mieux adaptée à des échantillons d'une formation superficielle, est en outre décrite dans la présente invention. Par ailleurs, la présente invention définit des corrections à appliquer aux paramètres TOC et MinC déterminés selon l'art antérieur, de manière à déterminer des teneurs en carbone organique et en carbone minéral qui soient plus proches des teneurs en carbone organique et en carbone minéral déterminées par des méthodes normalisées.

Ainsi, la présente invention permet une caractérisation rapide, simple et fiable des formes de carbone présentes dans un échantillon d'une formation superficielle, au moyen de paramètres standards corrigés.

**Résumé de l'invention**

La présente invention concerne un procédé pour caractériser et quantifier le carbone présent dans une formation superficielle, à partir d'un échantillon représentatif de ladite formation superficielle, dans lequel on applique au moins les étapes suivantes audit échantillon :

A) on chauffe ledit échantillon selon une première séquence de chauffe sous atmosphère inerte, et on mesure en continu une quantité de composés hydrocarbonés, une quantité de CO et une quantité de $CO_2$ libérés pendant ladite première séquence de chauffe, ladite première séquence de chauffe sous atmosphère inerte comprenant au moins un palier isotherme d'une durée prédéterminée à une température initiale (T0) suivi d'un gradient thermique pour atteindre une température finale (TF), ladite température initiale de pyrolyse (T0) étant comprise entre 80 et 200°C, ladite température finale de pyrolyse (TF) étant comprise entre 600 et 650°C ;

B) on chauffe un résidu dudit échantillon issu de ladite première séquence de chauffe selon une seconde séquence de chauffe sous atmosphère oxydante, et on mesure une quantité de CO et une quantité de $CO_2$ libérés pendant ladite seconde séquence de chauffe, ladite deuxième séquence de chauffe sous atmosphère oxydante débutant à une température initiale comprise entre 150 et 300°C, se terminant à une température finale comprise entre 850 et 1200°C, et suivant un gradient thermique ;

C) on détermine une valeur d'un paramètre SCmin représentatif d'une proportion en carbone minéral par rapport au carbone total dudit échantillon à partir d'un ratio entre ladite quantité de $CO_2$ libéré par ledit résidu dudit échantillon au-delà d'une température intermédiaire de ladite deuxième séquence de chauffe comprise entre 620 et 680°C, et ladite quantité de $CO_2$ libéré par ledit résidu pendant ladite deuxième séquence de chauffe ;

D) à partir desdites mesures desdites quantités de HC, de CO, et de CO2 libérés pendant lesdites première et deuxième séquences de chauffe, on détermine des paramètres définis selon des formules du type :

$$TOC \ = [S2 * 0.083] + \left[S3 * \frac{12}{440}\right] + \left[\left(S3CO + \frac{S3'CO}{2}\right) * \frac{12}{180}\right] + \left[S4CO_2 * \frac{12}{440}\right] + \left[S4CO * \frac{12}{280}\right]$$

$$MinC \ = \left[S3' * \frac{12}{440}\right] + \left[\frac{S3'CO}{2} * \frac{12}{280}\right] + \left[S5 * \frac{12}{440}\right]$$

où S2 représente ladite quantité de HC libérés pendant ladite première séquence de chauffe, S3 et S3' représentent lesdites quantités de $CO_2$ libérés pendant ladite première séquence de chauffe respectivement jusqu'à et au-delà d'une première température intermédiaire de ladite première séquence de chauffe comprise entre 370°C et 430°C, *S3CO* et *S3*'CO représentent lesdites quantités de CO libérés pendant ladite première séquence de chauffe respectivement jusqu'à et au-delà d'une deuxième température intermédiaire de ladite première séquence de chauffe comprise entre 520°C et 580°C, *S*4CO2 et *S*4CO représentent lesdites quantités respectivement de $CO_2$ et de CO libérés pendant ladite deuxième séquence de chauffe jusqu'à ladite température intermédiaire de ladite deuxième séquence de chauffe, et *S*5 représente ladite quantité de $CO_2$ libéré pendant ladite deuxième séquence de chauffe au-delà de ladite température intermédiaire de ladite deuxième séquence de chauffe ;

E) on quantifie une teneur en carbone organique et/ou une teneur en carbone minéral dudit échantillon de la manière suivante :

i) si ladite valeur dudit paramètre SCmin est inférieure ou égale à une valeur seuil prédéfinie dudit paramètre SCmin, ladite teneur en carbone minéral est nulle et ladite teneur en carbone organique est égale à une somme desdits paramètres TOC et MinC.

ii) si ladite valeur dudit paramètre SCmin est supérieure à ladite valeur seuil prédéfinie dudit paramètre SCmin :

- on détermine ladite teneur en carbone minéral Cmin selon une formule du type : ***Cmin** = MinC - kxTOC* ; et/ou

- on détermine ladite teneur en carbone organique Corg selon une formule du type : ***Corg** = TOC + kxTOC* où k est un facteur correctif compris entre 0.04 et 0.12.

Selon une mise en oeuvre de l'invention, ladite valeur seuil prédéfinie dudit paramètre SCmin peut être comprise entre 0.03 et 0.05, et vaut de préférence 0.04.

Selon une mise en oeuvre de l'invention, ledit facteur correctif k peut valoir préférentiellement 0.09.

Selon une mise en oeuvre de l'invention, ladite température initiale (T0) de la dite première séquence de chauffe peut être comprise entre 80°C et 150°C, et vaut de préférence 150°C.

Selon une mise en oeuvre de l'invention, ladite température initiale de ladite deuxième séquence de chauffe peut être égale à ladite température initiale (T0) de la dite première séquence de chauffe.

Selon une mise en oeuvre de l'invention, ladite première température intermédiaire de la dite première séquence de chauffe peut valoir 400°C.

Selon une mise en oeuvre de l'invention, ladite deuxième température intermédiaire de la dite première séquence de chauffe peut valoir 550°C.

Selon une mise en oeuvre de l'invention, ladite première séquence de chauffe peut comprendre un palier isotherme additionnel, à ladite température finale (TF) de la dite première séquence de chauffe.

Selon une mise en oeuvre de l'invention, ladite durée prédéterminée dudit ou desdits paliers isothermes de ladite première séquence de chauffe peut être comprise entre 3 et 5 minutes.

Selon une mise en oeuvre de l'invention, ledit gradient thermique de ladite première séquence de chauffe peut être compris entre 1 et 50°C.min$^{-1}$, de préférence entre 20 et 25°C.min$^{-1}$.

Selon une mise en oeuvre de l'invention, ledit gradient thermique de ladite deuxième séquence de chauffe peut être compris entre 20 et 40°C.min$^{-1}$, de préférence entre 20 et 25°C.min$^{-1}$

D'autres caractéristiques et avantages du procédé selon l'invention, apparaîtront à la lecture de la description ci-après d'exemples non limitatifs de réalisations, en se référant aux figures annexées et décrites ci-après.

**Liste des figures**

Les figures 1A et 1B illustrent de manière schématique respectivement la séquence de chauffe sous atmosphère inerte selon l'invention et une séquence de chauffe sous atmosphère inerte selon une mise en oeuvre de l'invention.

Les figures 2A et 2B illustrent des thermogrammes obtenus au moyen d'une mise en oeuvre de la séquence de chauffe sous atmosphère inerte selon l'invention appliquée à un échantillon de sol non carbonaté.

Les figures 2C et 2D illustrent des thermogrammes obtenus au moyen d'une mise en oeuvre de la séquence de chauffe sous atmosphère inerte selon l'invention appliquée à un échantillon de sol carbonaté.

Les figures 3A et 3B illustrent des thermogrammes obtenus au moyen d'une mise en oeuvre de la séquence de chauffe sous atmosphère oxydante selon l'invention, obtenus respectivement pour l'échantillon de sol non carbonaté des figures 2A et 2B et pour l'échantillon de sol carbonaté des figures 2C et 2D.

La figure 4 illustre l'évolution d'un paramètre MinC en fonction d'un paramètre TOC déterminés pour une pluralité d'échantillons correspondant à quatre classes d'abondance en carbone minéral.

**Description des modes de réalisation**

L'invention concerne un procédé pour caractériser et quantifier le carbone (ou autrement dit les formes de carbone) présentes dans une formation superficielle.

Par "formation superficielle" ("superficial deposit" en anglais), on entend une formation continentale ou littorale, meuble ou secondairement consolidée, provenant de la désagrégation mécanique et/ou chimique de roches préexistantes, et formée à l'interface lithosphère/biosphère/atmosphère. On distingue (i) les "formations superficielles allochtones" (comme les colluvions, alluvions, loess, etc.) qui ont subi ou subissent encore des déplacements proches ou lointains, et ne reposent plus sur leur matériel-parent, et les "formations superficielles autochtones" (comme les arènes, altérites, argiles à silex, etc.) qui ont évolué sur place à partir d'un matériel parent qui constitue encore leur substrat.

Par sol ("soil" en anglais), on entend l'ensemble des couches externes des formations superficielles, dont les propriétés sont directement contrôlées par les actions mutuelles de l'eau, de l'air et des organismes vivants et morts, voire des activités humaines pour les périodes les plus récentes.

Ces termes sont notamment définis dans le document de référence (Dictionnaire encyclopédique de Science du Sol, Mathieu & Lozet, Lavoisier, 2011).

Le procédé selon l'invention requiert de disposer d'au moins un échantillon représentatif de la formation superficielle : cet échantillon peut avoir été prélevé manuellement dans une fosse ou par carottage à l'aide d'une tarière. Avantageusement, l'échantillon tel que prélevé est tamisé au moyen d'un tamis dont les orifices ont un diamètre de 2 mm, séché à une température inférieure à 40°C, puis broyé jusqu'à obtenir des fragments ayant des dimensions inférieures à 200 $\mu$m.

Le procédé selon l'invention peut être avantageusement mais non limitativement mis en oeuvre au moyen du dispositif ROCK-EVAL® (IFP Energies nouvelles, France), tel que décrit dans les brevets FR 2227797 (US 3953171) et FR 2472754 (US 4352673). En effet, le dispositif ROCK-EVAL® comprend au moins :

- un four de pyrolyse en atmosphère non oxydante,

- des moyens de transfert des résidus de pyrolyse dans un four d'oxydation,

- un four d'oxydation en atmosphère oxydante,

- des moyens de mesure de la quantité de composés hydrocarbonés (HC) libérés au cours de la pyrolyse,

- des moyens de mesure du monoxyde de carbone (CO) et du dioxyde de carbone ($CO_2$).

Le procédé peut également être mis en oeuvre au moyen d'un unique four de pyrolyse, pouvant fonctionner à la fois en atmosphère non oxydante et en atmosphère oxydante, coopérant avec un dispositif de mesures de la quantité de composés hydrocarbonés libérés au cours de la pyrolyse, et un dispositif de mesure du monoxyde de carbone et du dioxyde de carbone.

Le procédé selon l'invention comprend au moins les étapes suivantes :

**1- Séquence de chauffe sous atmosphère inerte (pyrolyse)**

**2- Séquence de chauffe sous atmosphère oxydante (oxydation)**

**3- Caractérisation et quantification du carbone présent dans l'échantillon**

Les étapes du procédé selon l'invention sont détaillées ci-après de manière non limitative pour un échantillon de sol. Les étapes du procédé selon l'invention peuvent en effet tout aussi bien être appliquées à un échantillon provenant d'une autre couche d'une formation superficielle.

**1. Séquence de chauffe sous atmosphère inerte (pyrolyse)**

Au cours de cette étape, un échantillon de sol est chauffé sous atmosphère inerte (comme par exemple sous un flux d'azote, d'hélium) selon une séquence de températures prédéfinies, variables dans le temps.

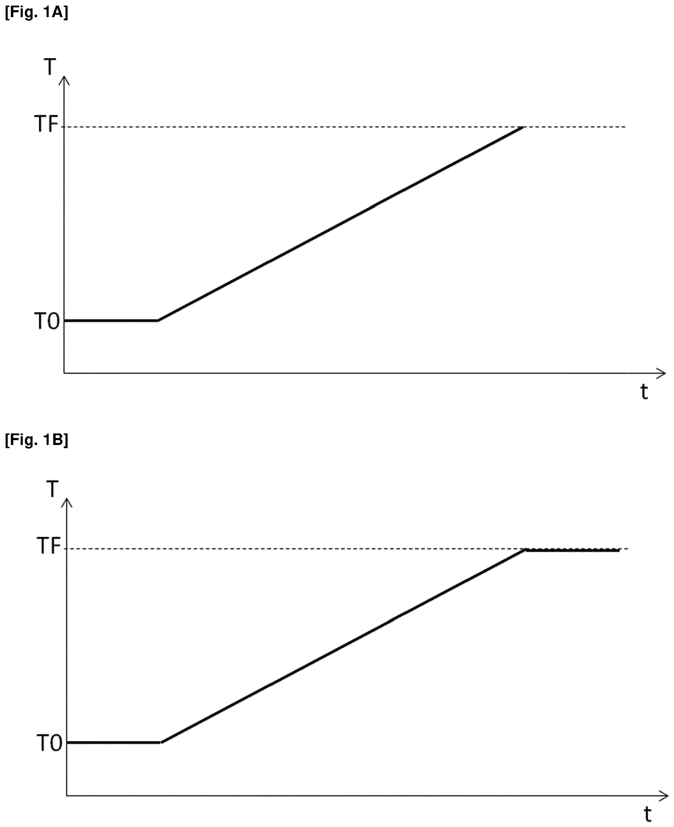

Selon l'invention, la séquence de chauffe sous atmosphère inerte comprend au moins un palier isotherme de durée non nulle à une température initiale (notée T0 par la suite) comprise entre 80 et 200°C, suivi par un gradient thermique prédéterminé de manière à élever la température jusqu'à une température finale (notée TF par la suite) comprise entre 600 et 650°C. Pour ce mode de réalisation, la séquence de chauffe peut comprendre de préférence uniquement un palier isotherme et un gradient thermique. La figure 1A illustre de manière schématique l'évolution de la température T en fonction du temps t d'une telle séquence de températures, présentant un palier isotherme à la température T0, suivi d'un gradient thermique jusqu'à la température TF.

De manière préférée, la séquence de chauffe sous atmosphère inerte comprend en outre un deuxième palier isotherme, à la température finale TF. Autrement dit, un deuxième palier isotherme à la température finale TF suit la phase de la séquence de chauffe se présentant sous la forme d'un gradient thermique. Cela permet de poursuivre, si besoin, le craquage des composés ayant une température de craquage proche de la température finale TF de la séquence de chauffe sous atmosphère inerte selon l'invention. La figure 1B illustre de manière schématique l'évolution de la température T en fonction du temps t d'une telle séquence de températures, présentant deux paliers isothermes, aux températures T0 et TF telles que définies ci-dessus. Pour ce mode de réalisation, la séquence de chauffe peut comprendre de préférence uniquement deux paliers isothermes et un gradient thermique.

Selon une mise en oeuvre de l'invention, le premier palier isotherme peut être de préférence à une température comprise entre 80°C et 150°C, de manière à permettre de récupérer les contributions des composés organiques les plus labiles présents dans un échantillon de sol. De préférence, le premier palier isotherme est à une valeur de 150°C, ce qui est une température suffisante pour récupérer les contributions des composés organiques les plus labiles présents dans la plupart des échantillons de sol.

Selon une mise en oeuvre de l'invention, la température finale TF vaut de préférence 600°C. Une telle température permet d'éviter d'obtenir des courbes de CO et de $CO_2$ présentant des pics incomplets en fin de pyrolyse, notamment lorsque la température maximale atteint 650°C, et en particulier sur des échantillons végétaux (litières, tourbes, composts).

Selon l'invention, la durée prédéterminée d'un palier isotherme est non nulle (par exemple supérieure à une demi-minute), et peut être préférentiellement comprise entre 3 et 5 minutes. De telles durées permettent de considérer que le craquage des composés ayant une température de craquage proche de la température du palier isotherme est terminé. Selon la mise en oeuvre de l'invention selon laquelle la séquence de chauffe sous atmosphère inerte selon l'invention comprend plusieurs paliers isothermes et en particulier deux paliers isothermes aux températures T0 et TF, la durée d'un palier isotherme peut être différente de la durée du ou des autres paliers isothermes.

Selon une mise en oeuvre de l'invention, le gradient thermique peut être compris entre 1 et 50°C.min$^{-1}$, de préférence être compris entre 20° et 25°C.min$^{-1}$. De telles valeurs constituent des compromis permettant le craquage thermique des composés, tout en limitant la durée de mise en oeuvre du procédé.

Selon l'invention, on mesure également, en continu, une quantité de composés hydrocarbonés libérés durant la chauffe sous atmosphère inerte, ainsi que la quantité de $CO_2$ et de CO contenus dans l'effluent résultant de ladite chauffe. Autrement dit, au cours de cette séquence, on mesure en continu la quantité de HC, de CO et de $CO_2$ libérés par l'échantillon par craquage thermique de la matière organique et par la décomposition thermique des minéraux carbonatés. Ainsi, on obtient à l'issue de cette étape appliquée à un échantillon donné, une première courbe représentative de la quantité de composés hydrocarbonés libérés au cours du temps pendant la phase de pyrolyse, ainsi que deux autres courbes représentatives de la quantité de CO et $CO_2$ libérés au cours du temps, pendant la phase de pyrolyse. La mesure de la quantité de composés hydrocarbonés peut être réalisée au moyen d'un détecteur du type à ionisation de flamme (FID). La mesure de la quantité de CO et $CO_2$ libérés peut être réalisée au moyen d'un détecteur du type infrarouge (IR). A noter que de tels capteurs mesurent un flux de HC, de CO et/ou de $CO_2$, et donnent des valeurs mesurées en millivolts (mV). De manière classique, on peut déterminer une quantité de HC, de CO et/ou de $CO_2$ en déterminant une aire sous la courbe mesurée (éventuellement entre des températures prédéfinies) par ces capteurs, et en divisant cette aire par la masse en mg de l'échantillon. En variante, d'autres moyens de mesure de la quantité de HC, de CO et/ou de $CO_2$ peuvent être utilisés.

De manière générale, cette séquence particulière de chauffe sous atmosphère inerte est suffisante pour permettre le craquage thermique des classes de composés comprenant du carbone minéral et du carbone organique, notamment :

- les composés très labiles thermiquement, qui sont particulièrement abondants dans les tissus biologiques frais, et qui sont en général libérés à des températures comprises entre environ 80 et 360°C ;
- les composés labiles thermiquement, qui sont majoritaires dans les échantillons organiques comme les litières ou les tourbes, et qui sont en général libérés à des températures comprises entre environ 360 et 420 °C ;
- les composés résistant thermiquement, qui sont majoritaires dans les échantillons organo-minéraux (sols) ou minéraux (alluvions, colluvions), et qui sont en général libérés à des températures comprises entre environ 420°C et 470 °C ;
- les composés réfractaires thermiquement, et qui sont en général libérés à des températures comprises entre environ 470 et 520 ;
- et les composés très réfractaires thermiquement, qui sont présents en plus grandes proportions dans des résidus de décomposition ou des fractions exogènes, comme les matières organiques pyrogéniques ou pétrogéniques, et qui sont en général libérés à des températures comprises entre environ 520 et 650°C.

Selon une mise en oeuvre de l'invention, la séquence de chauffe sous atmosphère inerte selon l'invention peut être précédée par une phase de montée en température du four de pyrolyse, qui peut être sous la forme d'un gradient thermique, par exemple compris entre 1 et 50°C.min$^{-1}$, de préférence entre 20° et 25°C.min$^{-1}$, ou de tout autre forme de courbe de montée en température du four de pyrolyse. Cette phase préliminaire de montée en température du four de pyrolyse permet de mettre le four de pyrolyse à la température du premier palier isotherme de la séquence de chauffe en atmosphère inerte selon l'invention. Cette phase préliminaire peut contribuer à débuter le craquage thermique des composés dont la température de craquage est inférieure à la température du premier palier isotherme, notamment dans le cas de tissus biologiques frais.

Selon une mise en oeuvre de l'invention, la séquence de chauffe sous atmosphère inerte selon l'invention peut être suivie par une phase d'abaissement de la température du four de pyrolyse, qui peut être sous la forme d'un gradient thermique, par exemple compris entre -1 et -50°C.min$^{-1}$, de préférence entre -20° et -25°C.min$^{-1}$, ou de tout autre forme de courbe de baisse en température du four de pyrolyse. Cette phase terminale d'abaissement de la température du four de pyrolyse permet, si besoin, de terminer le craquage thermique des composés associés au dernier palier isotherme de la séquence de chauffe sous atmosphère inerte selon l'invention.

Selon l'invention, on définit deux températures intermédiaires (notée TIP1 et TIP2 par la suite) comprises entre la température initiale T0 et la température finale TF de la séquence de chauffe en atmosphère inerte. Plus précisément, la première température intermédiaire de la séquence de chauffe en atmosphère inerte est comprise entre 370 et 430°C, et vaut préférentiellement 400°C. La deuxième température intermédiaire de la séquence de chauffe en atmosphère inerte est comprise entre 520 et 580°C, et vaut préférentiellement 550°C. Il s'agit des températures classiquement utilisées dans l'art antérieur pour définir des quantités respectives de $CO_2$ et de CO intervenant dans la détermination des paramètres standards TOC et MinC.

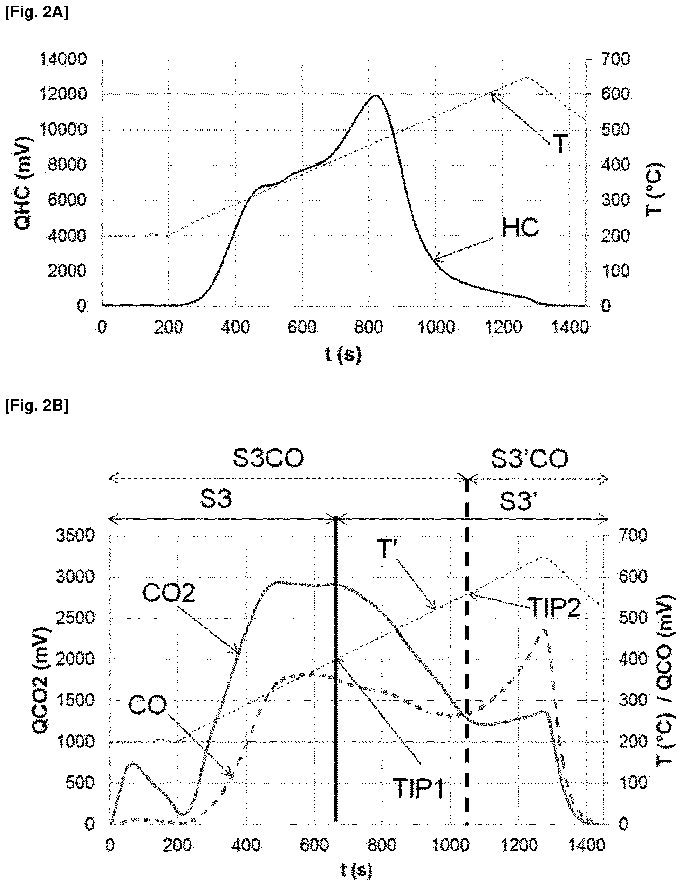

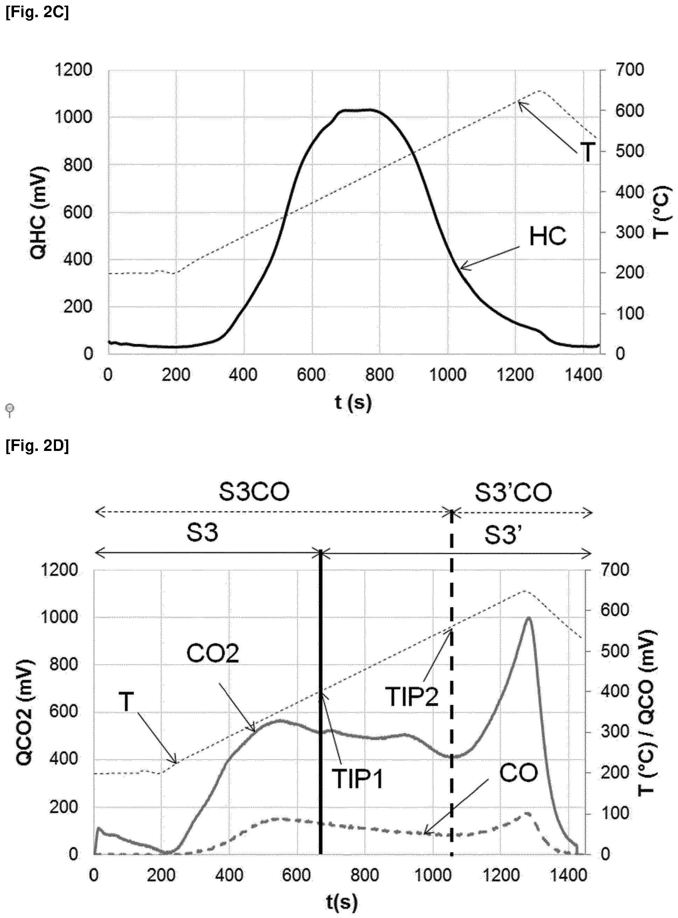

Les figures 2A et 2B (respectivement 2C et 2D) illustrent des thermogrammes obtenus au moyen d'une réalisation de la séquence de chauffe sous atmosphère inerte selon l'invention appliquée à un échantillon de sol non carbonaté (respectivement pour un sol carbonaté). Plus précisément, sur ces figures, la courbe T représente l'évolution en fonction du temps t de la température du four de pyrolyse pendant cette étape, ou autrement dit la courbe T représente la séquence de chauffe en atmosphère inerte à laquelle sont soumis les échantillons. On peut observer sur ces figures que la séquence de chauffe en atmosphère inerte mise en oeuvre pour ces échantillons débute par un premier palier à 200°C, suivi d'un gradient thermique qui permet d'élever la température jusqu'à une température finale de 650°C. Cette séquence de chauffe sous atmosphère inerte comprend une phase terminale d'abaissement de la température du four de pyrolyse. Par ailleurs, sur ces figures, la courbe HC (Figure 2A et 2C) représente l'évolution de l'intensité QHC (en mV) du signal d'un capteur FID mesurant la quantité de composés hydrocarbonés libérés pendant la séquence de chauffe en atmosphère inerte, la courbe CO (Figure 2B et 2D) représente l'évolution de l'intensité QCO (en mV) du signal d'un capteur IR mesurant la quantité de CO libéré pendant la séquence de chauffe en atmosphère inerte et la courbe CO2 (Figure 2B et 2D) représente l'évolution de l'intensité QCO2 (en mV) du signal d'un capteur IR mesurant la quantité de $CO_2$ libéré pendant la séquence de chauffe en atmosphère inerte. On peut observer que la courbe HC de la figure 2A présente un pic principal autour de 800s et un épaulement autour de 480s, alors que la courbe HC de la figure 2C présente un large pic autour de 780s, ce qui s'explique par les contributions relatives des classes de constituants craquant autour des températures correspondantes : les constituants thermiquement stables (craquage autour de 800s) étant relativement plus abondants dans le premier cas par rapport au second. De manière classique, on distingue deux portions dans la courbe de $CO_2$ libéré pendant la séquence de chauffe sous atmosphère inerte des figures 2B et 2D : une première portion à gauche de la première température intermédiaire TIP1, ici égale à 400°C, notée S3, qui est considérée classiquement (mais de manière erronée dans le cas de formations superficielles) comme correspondant au $CO_2$ ayant été généré par le craquage de la matière organique de l'échantillon pendant la chauffe sous atmosphère inerte, et une deuxième portion à droite de la première température intermédiaire TIP1, notée S3', qui est considérée classiquement (mais de manière erronée dans le cas de formations superficielles) comme correspondant au $CO_2$ généré par le craquage de la matrice minérale pendant la chauffe sous atmosphère inerte. On distingue également deux portions dans la courbe de CO libéré pendant la séquence de chauffe sous atmosphère inerte des figures 2B et 2D : une première portion à gauche de la deuxième température intermédiaire TIP2, ici sensiblement égale à 550°C, notée S3CO, qui est considérée classiquement (mais de manière erronée dans le cas de formations superficielles) comme correspondant au CO ayant été généré par le craquage de la matière organique de l'échantillon pendant la chauffe sous atmosphère inerte, et une deuxième portion à droite de cette deuxième température intermédiaire TIP2, notée S3'CO, qui est considérée classiquement comme correspondant à un mélange de CO généré par le craquage de la matière organique et par la décomposition thermique de la matrice minérale pendant la chauffe sous atmosphère inerte. On peut observer que les courbes $CO_2$ et CO de la figure 2B présentent un pic autour de 1260s peu marqué comparé à ceux observés sur les courbes CO2 et CO de la figure 2D ce qui s'explique par la contribution en $CO_2$ et en CO liée à la décomposition des carbonates dans l'échantillon de sol carbonaté.

**2) Séquence de chauffe sous atmosphère oxydante (oxydation)**

Au cours de cette deuxième étape, le résidu solide de l'échantillon obtenu à l'issue de la séquence de de chauffe sous atmosphère inerte telle que décrite à l'étape 1 ci-dessus est soumis à une oxydation selon un programme de températures prédéfinies, variables dans le temps.

Le programme de températures de la séquence de chauffe sous atmosphère oxydante selon l'invention est le suivant : à partir d'une température (notée T'min par la suite) comprise entre 150°C et 300°C, de préférence comprise entre 150°C et 250°C, et valant de manière préférentielle 150°C ou de manière très préférée étant égale à la température du premier palier isotherme de la séquence de chauffe en atmosphère inerte de manière à pouvoir effectuer des comparaisons, on élève la température du résidu de l'échantillon issu de l'étape 1) selon un gradient de température, de préférence compris entre 20 et 40°C.min$^{-1}$, très préférentiellement entre 20° et 25°C.min$^{-1}$, jusqu'à une température de fin d'oxydation (notée T'max par la suite) comprise entre 850 et 1200°C, et valant de préférence 900°C de manière à épuiser le stock de carbone minéral.

Selon l'invention, on mesure, en continu, des quantités représentatives de CO et de $CO_2$ libérés pendant cette seconde séquence de chauffe. Selon une mise en oeuvre de l'invention, cette mesure peut être réalisée au moyen d'un détecteur du type infrarouge (IR). A noter qu'un tel capteur mesure un flux de CO et/ou de $CO_2$, et fournit des valeurs mesurées en millivolts (mV). De manière classique, on détermine une quantité de CO et/ou de $CO_2$ en déterminant une aire sous la courbe mesurée (éventuellement entre des températures prédéfinies) par ce capteur, et on divise cette aire par la masse en mg de l'échantillon. En variante, d'autres moyens de mesure de la quantité de CO et/ou de $CO_2$ peuvent être utilisés.

De manière générale, la gamme de températures préférentielle pour la température initiale T'min de la séquence de chauffe en atmosphère oxydante, plus basse que les températures initiales connues de l'art antérieur (en général 300°C), permet d'éviter des épisodes de combustion instantanée du résidu de l'échantillon au début du cycle d'oxydation.

Selon l'invention, on définit une température intermédiaire (notée TLO par la suite) comprise entre la température initiale T'min et la température finale (T'max) de la séquence de chauffe en atmosphère oxydante, plus précisément comprise entre 620 et 680°C, et valant préférentiellement à 650 °C. Il s'agit d'une température classiquement utilisée dans l'art antérieur pour définir des quantités intervenant dans la détermination des paramètres standards TOC et MinC.

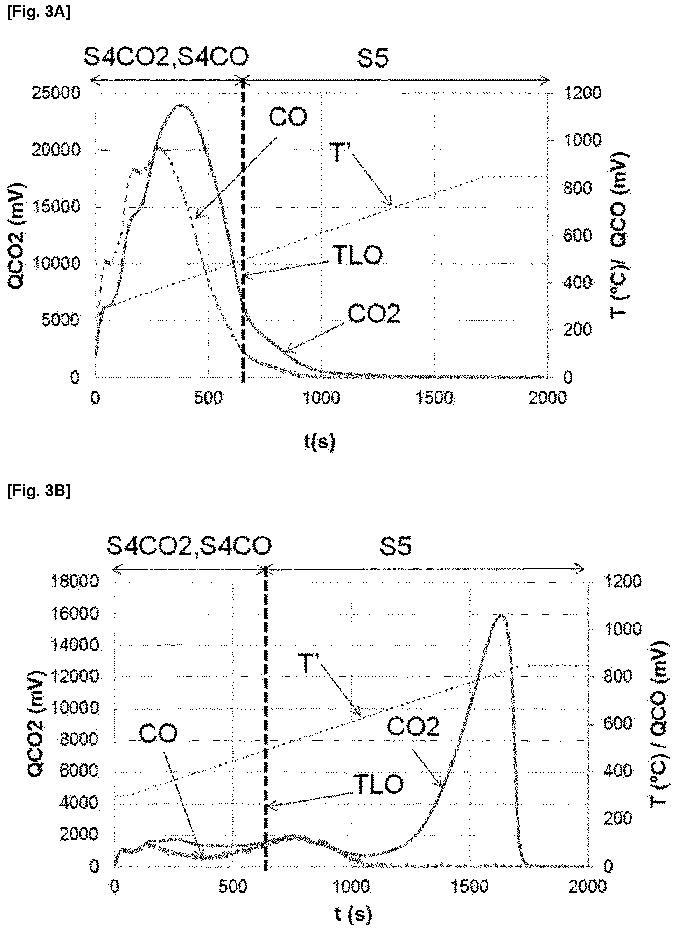

Les figures 3A et 3B illustrent des thermogrammes obtenus au moyen de la séquence de chauffe sous atmosphère oxydante selon l'invention, respectivement pour l'échantillon de sol non carbonaté des figures 2A et 2B et pour l'échantillon de sol carbonaté des figures 2B et 2D. Plus précisément, sur ces figures, la courbe T' représente l'évolution en fonction du temps t de la température du four d'oxydation pendant cette étape, ou autrement dit la courbe T' représente la séquence de chauffe en atmosphère oxydante à laquelle sont soumis les résidus des échantillons. Par ailleurs, la courbe CO2 (respectivement la courbe CO) représente l'évolution de l'intensité QCO2 en mV (respectivement QCO) du signal d'un capteur IR mesurant la quantité de $CO_2$ (respectivement CO) libéré au cours du temps par le résidu issu de l'étape 1) et soumis à la séquence de chauffe sous atmosphère oxydante T'. De manière classique, on appelle S4CO2 (respectivement S4CO) la portion de la courbe CO2 (respectivement courbe CO) à gauche de la température intermédiaire d'oxydation TLO, ici sensiblement égale à 650 °C, et qui est considérée classiquement comme correspondant au $CO_2$ (respectivement au CO) ayant été généré par le craquage de la matière organique du résidu de l'échantillon pendant la chauffe sous atmosphère oxydante. De manière classique, on appelle S5 la portion de la courbe CO2 à droite de la température intermédiaire d'oxydation TLO et qui est considérée classiquement comme correspondant au $CO_2$ ayant été généré par la décomposition des minéraux carbonatés du résidu de l'échantillon pendant la chauffe sous atmosphère oxydante. On peut observer sur ces figures 3A et 3B les flux de $CO_2$ au-dessus d'une température d'environ 650°C qui sont spécifiques aux sols carbonatés et témoignent de la présence de formes minérales de carbone. On peut aussi observer que la courbe CO s'achève dans les deux cas autour de la température intermédiaire TLO.

### 3) Caractérisation et quantification du carbone présent dans l'échantillon

A cours de cette étape, il s'agit de caractériser et de quantifier le carbone présent dans l'échantillon de sol, et notamment de déterminer une teneur en carbone organique et/ou une teneur en carbone minéral. Cette étape comprend au moins les deux sous-étapes détaillées ci-dessous.

### 3.1) Détermination d'un paramètre caractérisant la proportion en carbone minéral dans l'échantillon

Selon l'invention, cette première sous-étape vise à déterminer un paramètre caractérisant la proportion de carbone minéral par rapport au carbone total présent dans l'échantillon considéré, à partir de la quantité de $CO_2$ mesurée à l'étape 2 décrite ci-dessus.

Plus précisément, selon l'invention, on détermine un paramètre noté SCmin, représentatif d'une proportion en carbone minéral par rapport au carbone total dans ledit échantillon à partir d'un ratio entre la quantité de $CO_2$ libéré par le résidu de l'échantillon au-delà de la température intermédiaire TLO de la séquence de chauffe sous atmosphère oxydante décrite ci-dessus, et la totalité de la quantité de $CO_2$ libéré par ce résidu pendant la séquence de chauffe sous atmosphère oxydante. Ainsi, le paramètre SCmin est défini par la proportion de $CO_2$ libéré par décomposition thermique du carbone minéral contenu l'échantillon au cours de la séquence de chauffe en atmosphère oxydante par rapport à la quantité totale de $CO_2$ émise au cours de cette séquence de chauffe en atmosphère.

Selon une mise en oeuvre de l'invention, on peut déterminer le paramètre SCmin selon une formule du type :

$$SCmin = \frac{S5}{S4CO2 + S5}$$

où S5 et $S4CO2$ sont définis à l'étape précédente. De manière générale, le terme S5 correspond très majoritairement au $CO_2$ émis par décomposition des espèces minérales carbonatées et le terme $S4CO2$ correspond à la quantité de $CO_2$ émis par combustion du carbone organique résiduel de la pyrolyse au cours de la phase d'oxydation.

Selon l'invention, on définit une valeur seuil du paramètre SCmin en deçà de laquelle on peut considérer que l'échantillon de sol est dépourvu de forme minérale de carbone (c'est-à-dire que l'échantillon est non carbonaté), ou autrement dit, en deçà de laquelle on peut considérer que toute forme de carbone contenue dans cet échantillon est de nature organique.

Selon une mise en oeuvre de l'invention, la valeur seuil du paramètre Scmin, notée *SCmin_seuil,* selon laquelle l'échantillon de sol considéré est dépourvu de forme minérale peut être comprise entre 0.03 et 0.05, et vaut de préférence 0.04. Ces valeurs correspondent aux erreurs liées à la mise en oeuvre du procédé selon l'invention, dues à l'appareil de mesure lui-même et à la détermination des quantités de $CO_2$ libérées en deçà et au-delà de la température intermédiaire TLO définie à l'étape précédente.

Selon une mise en oeuvre de l'invention, on peut en outre caractériser le carbone d'un échantillon en définissant quatre classes d'abondance des formes minérales de carbone d'un échantillon, les classes d'abondance pouvant être définies en fonction de la valeur du paramètre SCmin selon l'invention de la manière suivante :

- Si SCmin < 0.04 , l'échantillon ne comporte pas de forme minérale de carbone ; on note cette classe SCmin1 par la suite ;

- Si 0.04 < SCmin < 0.2 : l'échantillon comporte des formes minérales de carbone sous forme de traces ; on note cette classe SCmin2 par la suite ;

- Si 0.2 < SCmin < 0.6 : l'échantillon comporte des formes minérales de carbone ; on note cette classe SCmin3 par la suite ;

- Si SCmin > 0.6 : l'échantillon comporte des formes minérales de carbone en abondance ; on note cette classe SCmin4 par la suite.

**3.2) Détermination d'une teneur en carbone organique et/ou une teneur en carbone minéral**

Au cours de cette étape, on détermine une teneur en carbone organique et/ou une teneur en carbone minéral à partir des quantités de HC, de CO et de $CO_2$ mesurées lors des étapes 1) et 2) décrites ci-dessus, et en fonction de la valeur du paramètre SCmin décrit à la sous-étape 3.1) précédente.

Selon l'invention, on détermine dans un premier temps les paramètres classiques TOC et MinC définis dans l'art antérieur, puis on détermine la teneur en carbone organique et/ou en carbone minéral à partir de corrections appliquées à ces paramètres classiques, car ils ne sont pas adaptés à des échantillons de formations superficielles. De plus, selon l'invention, les corrections à apporter aux paramètres standards sont fonction de la valeur du paramètre SCmin par rapport à la valeur seuil *SCmin_seuil* définie à la sous-étape précédente.

Selon l'invention, on détermine le paramètre TOC et le paramètre MinC tels que connus dans l'art antérieur, et notamment dans le document (Behar et al., 2001), selon des formules du type :

-

$$TOC \;\; = \; [S2 * 0.083] + \left[S3 * \tfrac{12}{440}\right] + \left[S3CO + \tfrac{S3'CO}{2}\right) * \tfrac{12}{180}\right] + \left[S4CO_2 * \tfrac{12}{440}\right] + \left[S4CO * \tfrac{12}{280}\right]$$

-

$$MinC \;\; = \; \left[S3' * \tfrac{12}{440}\right] + \left[\tfrac{S3'CO}{2} * \tfrac{12}{280}\right] + \left[S5 * \tfrac{12}{440}\right]$$

Selon l'invention, on définit deux cas possibles en fonction de la valeur du paramètre SCmin :

a) Premier cas : Scmin $\leq$ *SCmin_seuil*

Selon l'invention, si la valeur du paramètre SCmin est inférieure ou égale à la valeur seuil
*SCmin_seuil* définie à la sous-étape précédente (c'est-à-dire si l'échantillon considéré est non carbonaté), alors on définit :

- Une teneur en carbone minérale selon la formule suivante : **Cmin** = 0 ;

- Une teneur en carbone organique selon la formule suivante : **Corg** = *TOC + MinC ;*

Ainsi, contrairement à l'enseignement de l'art antérieur qui calculait un paramètre MinC non nul même dans le cas d'une absence de carbone minéral dans l'échantillon, la présente invention permet de déterminer une teneur en carbone minéral représentative de la teneur en carbone minéral réelle d'un échantillon d'une formation superficielle sans forme minérale de carbone. De plus, la teneur en carbone organique selon l'invention est déterminée en prenant en compte, contrairement à l'art antérieur, la totalité des quantités de CO et de $CO_2$ libérés pendant les séquences de chauffe en atmosphère inerte et en atmosphère oxydante. En effet, la détermination de la teneur en carbone organique selon l'art antérieur ne considérait que les quantités de CO et $CO_2$ libérés en deçà des températures intermédiaires TIP1 et TIP2 de la séquence de chauffe sous atmosphère inerte définies à l'étape 1), la moitié de la quantité de CO libéré au-delà de la température intermédiaire TIP2, et les quantités en CO et $CO_2$ libérés en deçà de la température intermédiaire TLO de la séquence de chauffe sous atmosphère oxydante définie à l'étape 2).

b) Deuxième cas : Scmin > *SCmin_seuil*

Selon l'invention, si la valeur du paramètre SCmin est supérieure à la valeur seuil *SCmin_seuil* définie à la sous-étape précédente (c'est-à-dire si l'échantillon considéré est carbonaté), alors on définit :

- Une teneur en carbone minérale selon la formule suivante : **Cmin** = *MinC - kxTOC*
- Une teneur en carbone organique selon la formule suivante : **Corg** = *TOC + kxTOC*

où k est un facteur correctif compris entre 0.04 et 0.12, et vaut préférentiellement 0.09.

Autrement dit, la teneur en carbone organique est égale à la somme du TOC et de la fraction organique thermiquement stable estimée comme valant entre 4 et 12% du TOC, préférentiellement 9%, et la teneur en carbone organique est égale au MinC moins cette fraction thermiquement stable de nature organique.

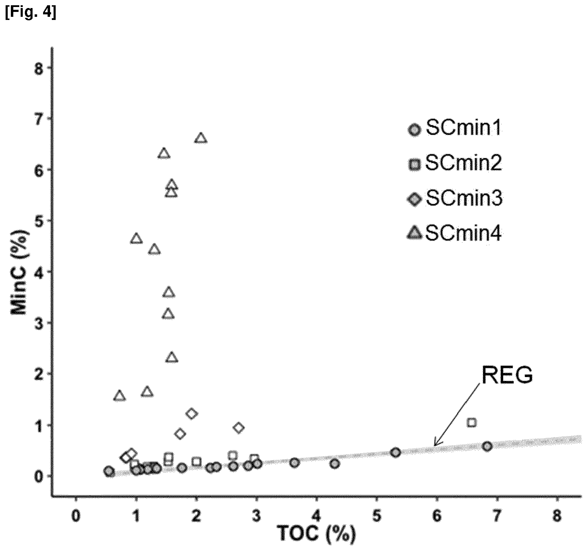

Ces corrections ont été établies à partir d'une pluralité d'échantillons (environ 50) provenant de formations superficielles ayant des proportions en carbone organique et minéral très variées, plus précisément pour des valeurs du paramètres SCmin variant dans les 4 classes d'abondance SCmin1 à SCmin4 décrites ci-dessus. Pour chacun de ces échantillons, on a déterminé les paramètres classiques TOC et MinC selon l'art antérieur définis ci-dessus. La figure 4 présente l'évolution du paramètre MinC en fonction du paramètre TOC déterminés selon l'art antérieur pour la pluralité d'échantillons. On peut observer sur cette figure une très forte corrélation linéaire (droite REG) entre les paramètres TOC et MinC pour les échantillons des classes SCmin1 et SCmin2. En revanche, la dispersion est beaucoup plus importante pour les échantillons dans lesquels les formes minérales de carbone sont présentes ou abondantes (classe SCmin3), jusqu'à ce que la relation ne soit plus significative pour les échantillons de la classe SCmin 4. Une régression linéaire appliquée pour chacune des classes SCmin1 à Scmin4 conduit aux relations suivantes entre les paramètres TOC et MinC :

$$SCmin1 : \quad MinC = 0.09 \, x \, TOC \, , \quad avec \, R^2 = 0.96 \, et \, p < 4.10^{-11}$$

$$SCmin2 : \quad MinC = 0.15 \, x \, TOC \quad avec \, R^2 = 0.93 \, et \, p < 1.10^{-9}$$

$$SCmin3: \quad MinC = 0.42 \, x \, TOC \quad avec \, R^2 = 0.77 \, et \, p < 0.002$$

$$SCmin4 : \quad MinC = 3.03 \, x \, TOC \, , \quad avec \, R^2 = 0.35 \, et \, p > 0.05.$$

où $R^2$ correspond au coefficient de détermination de la régression linéaire et p correspond à une probabilité d'obtenir la même valeur ou une valeur encore plus extrême que celle observée.

La relation établie pour la catégorie SCmin1 traduit le fait que, dans ces échantillons sans forme minérale de carbone, l'ensemble du carbone comptabilisé dans le MinC est une fraction thermiquement stable du carbone organique. En outre, bien que le panel d'échantillons couvre des situations très variées, la dispersion autour de la droite de régression est très faible ($R^2 = 0.96$ et $p < 4.10^{-11}$), ce qui indique que la fraction organique thermiquement stable est identique dans tous les sols (autour de 9% du TOC). Elle peut donc être considérée comme une propriété générale, indépendante des contextes pédogénétiques. Pour les autres classes d'abondance en carbone minéral SCmin2, SCmin3, et SCmin4, il est raisonnable de penser que cette propriété est vérifiée, mais qu'elle est de plus en plus masquée par la part croissante des formes minérales de carbone, comme le témoignent l'augmentation concomitante du coefficient directeur de la droite de régression et de la dispersion du nuage de point. Il est donc légitime de généraliser en première approximation la relation établie pour la classe SCmin1 à toutes les classes d'abondance en carbone minéral des formations superficielles.

Ainsi, les définitions des teneurs en carbone minéral et en carbone organique selon l'invention tiennent compte des formes de carbone présentes de la manière suivante : (i) toutes les formes de carbone sont organiques dans les échantillons tels que *SCmin ≤ SCmin_seuil* et (ii) les autres classes d'abondance de carbone minéral comprennent une part de carbone organique thermiquement stable à laquelle s'ajoute une part de carbone minéral.

Ainsi, à l'issue de ces deux sous-étapes, on obtient une caractérisation et une quantification fiable du carbone présent dans un échantillon d'une formation superficielle, via la détermination d'un paramètre SCmin représentant une proportion en carbone minéral dans l'échantillon, et la détermination de la teneur en carbone organique et/ou en carbone minéral de l'échantillon à partir de corrections apportées aux paramètres TOC et MinC déterminés selon l'art antérieur.

### 3.3) Détermination du statut thermique des formes organiques de carbone

Au cours de cette sous-étape, qui est optionnelle, il s'agit de déterminer le statut thermique des formes organiques de carbone de l'échantillon. Par statut thermique des formes organiques de carbone présentes dans l'échantillon, on entend un indicateur de la répartition des différentes classes de composés présentes dans l'échantillon et définies chacune par leur température de craquage.

La matière organique d'un sol est un mélange hétérogène complexe comprenant des constituants de nature et d'origine diverses : résidus de la décomposition graduelle des constituants biogéniques les plus labiles, particules libres et constituants impliqués dans les complexes organo-minéraux, constituants pyrogéniques ou pétrogéniques. Le procédé de fractionnement thermique selon l'invention ne permet pas de séparer ces constituants spécifiques. En revanche, il permet de mesurer les contributions des classes de composés définies chacune par leur température de craquage. La stabilité thermique étant considérée comme une variable liée à la stabilité biogéochimique (i.e. la résistance à la décomposition par les microorganismes), les contributions des classes de composés ainsi définies peuvent être utilisées pour décrire l'hétérogénéité de la matière organique des sols.

Par la suite, nous allons introduire les notations définies dans les tableaux 1 et 2 ci-dessous : Par la suite, on peut utiliser les paramètres définis dans le tableau 1 ci-après. Plus précisément, les paramètres $S2_i$ (respectivement $S3CO2_i$ et $S3CO_i$), avec i variant de 0 à 6 correspondent aux quantités de HC (respectivement $CO_2$ et CO) libérés pendant les gammes de températures $DT_i$ définies de la manière suivante : $DT_0 = T0-$ ; $T0 < DT_1 \leq T1$ ; $T1 < DT_2 \leq T2$ ; $T2 < DT_3 \leq T3$ ; $T3 < DT_4 \leq T4$ ; $T4 < DT_5 \leq T5$ ; $DT_6 = TF+$ ; et où T0- correspond au premier palier isotherme à la température T0 de la séquence de chauffe en atmosphère inerte, éventuellement précédé d'une phase préliminaire de montée en température du four de pyrolyse ; et où TF+ correspond à la température finale TF de la séquence de chauffe en atmosphère inerte, éventuellement suivi d'une phase terminale de palier isotherme à la température TF, ou encore d'une phase d'abaissement de la température du four de pyrolyse, et où T1 est une température intermédiaire comprise entre 320 et 360°C, T2 est une température intermédiaire comprise entre 400 et 440°C (alternativement, comprise entre 380 et 420°C), T3 est une température intermédiaire comprise entre 380 et 420°C (alternativement, comprise entre 440 et 480°C), T4 est une température intermédiaire comprise entre 440 et 480°C (alternativement, comprise entre 500 et 540°C), et T5 est une température intermédiaire comprise entre 500 et 540°C (alternativement, comprise entre 600 et 650°C). Ces températures intermédiaires correspondent à des minimas relatifs observés sur des courbes de HC, de CO et de $CO_2$ mesurées pour une pluralité d'échantillons de formations superficielles, et notamment de sols, de nature et d'origine variées.

[Table 1]

| Température | Mesure HC | Mesure $CO_2$ | Mesure CO |
|---|---|---|---|
| $DT_0 = T0-$ | $S2_0$ | $S3CO2_0$ | $S3CO_0$ |
| $T0 < DT_1 \leq T1$ | $S2_1$ | $S3CO2_1$ | $S3CO_1$ |
| $T1 < DT_2 \leq T2$ | $S2_2$ | $S3CO2_2$ | $S3CO_2$ |
| $T2 < DT_3 \leq T3$ | $S2_3$ | $S3CO2_3$ | $S3CO_3$ |
| $T3 < DT_4 \leq T4$ | $S2_4$ | $S3CO2_4$ | $S3CO_4$ |
| $T4 < DT_5 \leq T5$ | $S2_5$ | $S3CO2_5$ | $S3CO_5$ |
| $DT_6 = T5+$ | $S2_6$ | $S3CO2_6$ | $S3CO_6$ |

Par la suite, on peut utiliser en outre les paramètres définis dans le tableau 2 ci-après. Plus précisément, les paramètres $S4CO2_i$ et $S4CO_i$, avec i variant de 0 à 4, correspondent aux quantités de $CO_2$ et CO libérés pendant les gammes de températures $DT'_i$ définies de la manière suivante : $DT'0 \leq T'min$ ; $T'min < DT'1 \leq T'inti$ ; $T'inti < DT'2 \leq T'int2$ ; $T'int2 < DT'3 \leq T'int3$ et $T'int3 < DT'4 \leq T'max$, où T'int1, T'int2, et T'int3 sont des températures intermédiaires comprises entre T'min et T'max et sont telles que T'int1 est comprise entre 420 et 480°C et vaut de préférence 460°C, T'int2 est comprise entre 520 et 580°C et vaut de préférence 550°C, et T'int3 est comprise entre 630 et 670°C et vaut de préférence 650°C. Ces températures intermédiaires correspondent à des minimas relatifs observés par la demanderesse sur des courbes de CO et de $CO_2$ mesurées pour une pluralité d'échantillons de formations superficielles, et notamment de sols, de nature et d'origine variées. Considérant que, selon la littérature, la température de combustion est une approximation de la stabilité thermique, les flux de CO et $CO_2$ mesurés dans les gammes de température DT'1 , DT'2 et DT'3 peuvent être rapportés à des classes de constituants organiques de stabilité croissante. Considérant que la limite de stabilité thermique de la calcite est proche de T'int3, les flux de $CO_2$ mesurés dans la gamme de température DT'4 peuvent être rapportés à des formes minérales de carbone.

[Table 2]

| | | |
|---|---|---|
| $DT'_0 \leq T'min$ | $S4CO2_0$ | $S4CO_0$ |
| $T'min < DT'_1 \leq T'int1$ | $S4CO2_1$ | $S4CO_1$ |
| $T'int1 < DT'_2 \leq T'int2$ | $S4CO2_2$ | $S4CO_2$ |
| $T'int2 < DT'_3 \leq T'int3$ | $S4CO2_3$ | $S4CO_3$ |

(suite)

| T'int3 <DT'4 $\leq$ T'max | S4CO2$_4$ | S4CO$_4$ |
|---|---|---|

Selon une première réalisation de cette variante de l'invention comprenant une sous-étape de détermination du statut thermique des formes de carbone de l'échantillon, on peut définir un indice de décomposition ID et un indice de stabilité IS à partir des contributions des classes de composés définies ci-dessus selon des formules du type :

- Indice de décomposition $\quad ID = \log [S2_1 + S2_2] / S2_3$
- Indice de stabilité $\quad IS = [S2_3 + S2_4 + S2_5 + S2_6] / 100$

Ainsi, ces deux indices sont directement liés à la fraction la plus réactive du carbone organique (c'est-à-dire la part de carbone pyrolysé sous forme de composés hydrocarbonés).

L'indice de stabilité IS mesure les contributions relatives des classes de composés thermiquement stables ($S2_3$, $S2_4$, $S2_5$ et $S2_6$) qui sont particulièrement abondantes dans les formations superficielles et les couches profondes des sols, par opposition aux classes de composés plus labiles ($S2_1$ et $S2_2$) et plus abondantes dans les tissus végétaux peu décomposés présents dans les couches organiques et les couches superficielles des sols.

L'indice de décomposition ID mesure le rapport entre ces classes de composés les plus labiles ($S2_1$ et $S2_2$) et la classe de composés intermédiaire ($S2_3$) qui est particulièrement abondante dans les couches organiques et organo-minérales des sols. De manière générale, l'indice de décomposition ID mesure le degré de transformation de la matière organique au fur et à mesure que les composés des classes de composés les plus labiles sont décomposés et que les composés des classes de composés les plus stables s'accumulent.

Selon une deuxième réalisation de cette variante de l'invention comprenant une sous-étape de détermination du statut thermique des formes de carbone de l'échantillon, on détermine le statut thermique des formes de carbone d'un échantillon à partir des contributions relatives des différentes classes de composés d'un échantillon pour calculer une teneur en carbone organique $C_{org}$ thermiquement labile (c'est-à-dire résultant du craquage thermique et de la combustion en-dessous de T3) et une teneur en carbone organique $C_{org}$ thermiquement stable (c'est-à-dire résultant du craquage thermique et de la combustion en-dessous de T3).

Selon une mise en oeuvre de l'invention, on peut déterminer au moins un des paramètres caractérisant le statut thermique de l'échantillon suivants :

- une teneur en carbone organique pyrolysé thermiquement labile, notée COPL, définie selon une formule du type :

$$COPL\ (\%C) = (S2_L \times 0.083) + ([KCOPL * S3CO2_L] \times \frac{12}{440}) + (S3CO_L \times \frac{12}{280})$$

avec KCOPL compris entre 1 et 2, et valant préférentiellement 1.3359.

- une teneur en carbone organique pyrolysé thermiquement stable, notée COPS, définie selon une formule du type :

$$COPS\ (\%C) = (S2_S \times 0.083) + ([KCOPS * S3CO2_L] \times \frac{12}{440}) + ([½ S3CO_S] \times \frac{12}{280})$$

avec KCOPS compris entre 0.1 et 1, et valant préférentiellement 0.6274.

- une teneur en carbone organique résiduel thermiquement labile, notée CORL, définie selon une formule du type :

$$CORL\ (\%C) = (S4CO2_L \times \frac{12}{440}) + (S4CO_L \times \frac{12}{280})$$

- une teneur en carbone organique résiduel thermiquement stable, notée CORS, définie selon une formule du type :

$$CORS\ (\%C) = (S4CO2_S \times \frac{12}{440})$$

Où

$$S2_L = \sum_{i=0}^{i=3} S2_i; \qquad S2_S = \sum_{i=4}^{i=6} S2_i$$

$$S3CO2_L = \sum_{i=0}^{i=3} S3CO2_i \quad ; \quad S3CO2_S = \sum_{i=4}^{i=6} S3CO2_i \quad ;$$

$$S3CO_L = \sum_{i=0}^{i=4} S3CO_i \quad ; \quad S3CO_S = \sum_{i=5}^{i=6} S3CO_i \quad ;$$

$$S4CO2_L = \sum_{i=0}^{i=1} S4CO2_i \quad ; \quad S4CO2_S = \sum_{i=2}^{i=3} S4CO2_i \quad ;$$

$$S4CO_L = \sum_{i=0}^{i=1} S4CO_i \quad ; \quad S4CO_S = \sum_{i=2}^{i=3} S4CO_i \quad .$$

Ces teneurs partielles sont particulièrement utiles pour comprendre la dynamique de la matière organique dans les sols car elles permettent de comparer les stocks totaux de carbone organique avec les contributions des différentes formes de carbone, et d'effectuer un suivi de l'évolution de ces stocks au cours du temps. Elles permettent notamment d'explorer les relations entre les stocks en carbone organique thermiquement labile et en en carbone organique thermiquement stable qui sont directement corrélés dans les sols, ce qui indique que le transfert d'un stock à l'autre s'opère effectivement dans les sols.

Ainsi, la présente invention permet la caractérisation et la quantification de manière simple, rapide et fiable des formes de carbone présentes dans un échantillon d'une formation superficielle, que la formation superficielle soit carbonatée ou non. En particulier, la présente invention définit des teneurs en carbone organique et/ou en carbone minéral prenant en compte une quantité estimée de $CO_2$ libéré par craquage thermique de la matière organique de l'échantillon pendant la séquence de chauffe en atmosphère inerte au-delà d'une température qui était considérée comme une température intermédiaire dans l'art antérieur.

## Revendications

1. Procédé pour caractériser et quantifier le carbone présent dans une formation superficielle, à partir d'un échantillon représentatif de ladite formation superficielle, **caractérisé en ce qu'**on applique au moins les étapes suivantes audit échantillon :

A) on chauffe ledit échantillon selon une première séquence de chauffe sous atmosphère inerte, et on mesure en continu une quantité de composés hydrocarbonés, une quantité de CO et une quantité de $CO_2$ libérés pendant ladite première séquence de chauffe, ladite première séquence de chauffe sous atmosphère inerte comprenant au moins un palier isotherme d'une durée prédéterminée à une température initiale (T0) suivi d'un gradient thermique pour atteindre une température finale (TF), ladite température initiale de pyrolyse (T0) étant comprise entre 80 et 200°C, ladite température finale de pyrolyse (TF) étant comprise entre 600 et 650°C ;
B) on chauffe un résidu dudit échantillon issu de ladite première séquence de chauffe selon une seconde séquence de chauffe sous atmosphère oxydante, et on mesure une quantité de CO et une quantité de $CO_2$ libérés pendant ladite seconde séquence de chauffe, ladite deuxième séquence de chauffe sous atmosphère oxydante débutant à une température initiale comprise entre 150 et 300°C, se terminant à une température finale comprise entre 850 et 1200°C, et suivant un gradient thermique ;
C) on détermine une valeur d'un paramètre SCmin représentatif d'une proportion en carbone minéral par rapport au carbone total dudit échantillon à partir d'un ratio entre ladite quantité de $CO_2$ libéré par ledit résidu dudit échantillon au-delà d'une température intermédiaire de ladite deuxième séquence de chauffe comprise entre 620 et 680°C, et ladite quantité de $CO_2$ libéré par ledit résidu pendant ladite deuxième séquence de chauffe ;
D) à partir desdites mesures desdites quantités de HC, de CO, et de CO2 libérés pendant lesdites première et deuxième séquences de chauffe, on détermine des paramètres définis selon des formules du type :

$$TOC = [S2 * 0.083] + \left[S3 * \frac{12}{440}\right] + \left[\left(S3CO + \frac{S3'CO}{2}\right) * \frac{12}{180}\right] + \left[S4CO_2 * \frac{12}{440}\right]$$
$$+ \left[S4CO * \frac{12}{280}\right]$$

$$MinC \;=\; \left[S3' * \frac{12}{440}\right] + \left[\frac{S3'CO}{2} * \frac{12}{280}\right] + \left[S5 * \frac{12}{440}\right]$$

où S2 représente ladite quantité de HC libérés pendant ladite première séquence de chauffe, S3 et S3' représentent lesdites quantités de $CO_2$ libérés pendant ladite première séquence de chauffe respectivement jusqu'à et au-delà d'une première température intermédiaire de ladite première séquence de chauffe comprise entre 370°C et 430°C, *S3CO* et *S3'CO* représentent lesdites quantités de CO libérés pendant ladite première séquence de chauffe respectivement jusqu'à et au-delà d'une deuxième température intermédiaire de ladite première séquence de chauffe comprise entre 520°C et 580°C, *S4CO2* et *S4CO* représentent lesdites quantités respectivement de $CO_2$ et de CO libérés pendant ladite deuxième séquence de chauffe jusqu'à ladite température intermédiaire de ladite deuxième séquence de chauffe, et 55 représente ladite quantité de $CO_2$ libéré pendant ladite deuxième séquence de chauffe au-delà de ladite température intermédiaire de ladite deuxième séquence de chauffe.

E) on quantifie une teneur en carbone organique et/ou une teneur en carbone minéral dudit échantillon de la manière suivante :

i) si ladite valeur dudit paramètre SCmin est inférieure ou égale à une valeur seuil prédéfinie dudit paramètre SCmin, ladite teneur en carbone minéral est nulle et ladite teneur en carbone organique est égale à une somme desdits paramètres TOC et MinC.

ii ) si ladite valeur dudit paramètre SCmin est supérieure à ladite valeur seuil prédéfinie dudit paramètre SCmin :

on détermine ladite teneur en carbone minéral Cmin selon une formule du type : ***Cmin** = MinC - kxTOC* ;

et/ou on détermine ladite teneur en carbone organique Corg selon une formule du type : ***Corg** = TOC + kxTOC* où k est un facteur correctif compris entre 0.04 et 0.12.

2. Procédé selon la revendication 1, dans lequel ladite valeur seuil prédéfinie dudit paramètre SCmin est comprise entre 0.03 et 0.05, et vaut de préférence 0.04.

3. Procédé selon l'une des revendications précédentes, dans lequel ledit facteur correctif k vaut préférentiellement 0.09.

4. Procédé selon l'une des revendications précédentes, dans lequel ladite température initiale (T0) de la dite première séquence de chauffe est comprise entre 80°C et 150°C, et vaut de préférence 150°C.

5. Procédé selon l'une des revendications précédentes, dans lequel ladite température initiale de ladite deuxième séquence de chauffe est égale à ladite température initiale (T0) de la dite première séquence de chauffe.

6. Procédé selon l'une des revendications précédentes, dans lequel ladite première température intermédiaire de la dite première séquence de chauffe vaut 400°C.

7. Procédé selon l'une des revendications précédentes, dans lequel ladite deuxième température intermédiaire de la dite première séquence de chauffe vaut 550°C.

8. Procédé selon l'une des revendications précédentes, dans lequel ladite première séquence de chauffe comprend un palier isotherme additionnel, à ladite température finale (TF) de la dite première séquence de chauffe.

9. Procédé selon l'une des revendications précédentes, dans lequel ladite durée prédéterminée dudit ou desdits paliers isothermes de ladite première séquence de chauffe est comprise entre 3 et 5 minutes.

10. Procédé selon l'une des revendications précédentes, dans lequel ledit gradient thermique de ladite première séquence de chauffe est compris entre 1 et 50°C.min$^{-1}$, de préférence entre 20 et 25°C.min$^{-1}$.

11. Procédé selon l'une des revendications précédentes, dans lequel ledit gradient thermique de ladite deuxième séquence de chauffe est compris entre 20 et 40°C.min$^{-1}$, de préférence entre 20 et 25°C.min$^{-1}$.

**Patentansprüche**

1. Verfahren zum Charakterisieren und Quantifizieren des Kohlenstoffs, welcher in einer oberflächennahen Formation vorliegt, ausgehend von einer Probe, die repräsentativ für die oberflächennahe Formation ist, **dadurch gekennzeichnet, dass** die Probe zumindest den folgenden Schritten unterzogen wird:

   A) Erhitzen der Probe gemäß einer ersten Erhitzungssequenz unter Inertgasatmosphäre, und kontinuierliches Messen einer Menge an Kohlenwasserstoffverbindungen, einer Menge an CO und einer Menge an $CO_2$, wie sie während der ersten Erhitzungssequenz freigesetzt werden, wobei die erste Erhitzungssequenz unter Inertgasatmosphäre zumindest eine isotherme Stufe von vorbestimmter Dauer bei einer Anfangstemperatur (T0), gefolgt von einem Temperaturgradienten umfasst, um eine Endtemperatur (TF) zu erreichen, wobei die Pyrolyse-Anfangstemperatur (T0) im Bereich von 80 bis 200 °C liegt, wobei die Pyrolyse-Endtemperatur (TF) im Bereich von 600 bis 650 °C liegt;
   B) Erhitzen eines Rückstands der Probe, der aus der ersten Erhitzungssequenz hervorgegangen ist, gemäß einer zweiten Erhitzungssequenz unter oxidierender Atmosphäre, und Messen einer Menge an CO und einer Menge an $CO_2$, wie sie während der zweiten Erhitzungssequenz freigesetzt werden, wobei die zweite Erhitzungssequenz unter oxidierender Atmosphäre bei einer Anfangstemperatur im Bereich von 150 bis 300 °C beginnt, bei einer Endtemperatur im Bereich von 850 bis 1200 °C endet und einem Temperaturgradienten folgt;
   C) Bestimmen eines Wertes eines Parameters SCmin, welcher für einen Anteil an mineralischem Kohlenstoff bezogen auf den Gesamtkohlenstoff der Probe steht, ausgehend von einem Verhältnis zwischen der Menge an $CO_2$, die von dem Rückstand der Probe jenseits eines Temperaturzwischenwerts der zweiten Erhitzungssequenz freigesetzt wird, welcher im Bereich von 620 bis 680 °C liegt, und der Menge an $CO_2$, die von dem Rückstand während der zweiten Erhitzungssequenz freigesetzt wird;
   D) ausgehend von den Messungen der Mengen an HC, an CO und an $CO_2$, wie sie während der ersten und der zweiten Erhitzungssequenz freigesetzt werden, Bestimmen von Parametern, die anhand von Formeln folgender Art definiert sind:

$$TOC = [S2 * 0{,}083] + [S3 * \frac{12}{440}] + [(S3CO + \frac{S3'CO}{2}) * \frac{12}{180}] + [S4CO_2 * \frac{12}{440}] + [S4CO * \frac{12}{280}]$$

$$MinC = [S3' * \frac{12}{440}] + [\frac{S3'CO}{2} * \frac{12}{280}] + [S5 * \frac{12}{440}]$$

   wobei S2 für die Menge an HC steht, welche während der ersten Erhitzungssequenz freigesetzt werden, S3 und S3' für die Mengen an $CO_2$ stehen, wie sie während der ersten Erhitzungssequenz bis zu einem beziehungsweise jenseits eines ersten Zwischentemperaturwert(s) der ersten Erhitzungssequenz, welcher im Bereich von 370 °C bis 430 °C liegt, freigesetzt werden, *S3CO* und *S3'CO* für die Mengen an CO stehen, wie sie während der ersten Erhitzungssequenz bis zu einem beziehungsweise jenseits eines zweiten Temperaturzwischenwert(s) der ersten Erhitzungssequenz, welcher im Bereich von 520 °C bis 580 °C liegt, freigesetzt werden, *S4CO2* und *S4CO* für die Mengen an $CO_2$ beziehungsweise an CO stehen, wie sie während der zweiten Erhitzungssequenz bis zum Temperaturzwischenwert der zweiten Erhitzungssequenz freigesetzt werden, und S5 für die Menge an $CO_2$ steht, die während der zweiten Erhitzungssequenz jenseits des Temperaturzwischenwerts der zweiten Erhitzungssequenz freigesetzt wird.
   E) Quantifizieren eines Gehalts an organischem Kohlenstoff und/oder eines Gehalts an mineralischem Kohlenstoff der Probe auf folgende Art und Weise:

   i) wenn der Wert des Parameters SCmin kleiner oder gleich einem vorbestimmten Schwellenwert für den Parameter SCmin ist, beträgt der Gehalt an mineralischem Kohlenstoff null, und der Gehalt an organischem Kohlenstoff ist gleich einer Summe aus den Parametern TOC und MinC.
   ii) wenn der Wert des Parameters SCmin höher als der vorbestimmte Schwellenwert für den Parameter SCmin ist: Bestimmen des Gehalts an mineralischem Kohlenstoff Cmin gemäß einer Formel folgender Art: *Cmin = MinC - kxTOC*; und/oder Bestimmen des Gehalts an organischem Kohlenstoff Corg gemäß einer Formel folgender Art: *Corg = toc + kxTOC,* wobei k ein Korrekturfaktor im Bereich von 0,04 bis 0,12 ist.

**2.** Verfahren nach Anspruch 1, wobei der vorbestimmte Schwellenwert für den Parameter SCmin im Bereich von 0,03 bis 0,05 liegt und vorzugsweise gleich 0,04 ist.

**3.** Verfahren nach einem der vorhergehenden Ansprüche, wobei der Korrekturfaktor vorzugsweise gleich 0,09 ist.

**4.** Verfahren nach einem der vorhergehenden Ansprüche, wobei die Anfangstemperatur (T0) der ersten Erhitzungssequenz im Bereich von 80 °C bis 150 °C liegt und vorzugsweise gleich 150 °C ist.

**5.** Verfahren nach einem der vorhergehenden Ansprüche, wobei die Anfangstemperatur der zweiten Erhitzungssequenz gleich der Anfangstemperatur (T0) der ersten Erhitzungssequenz ist.

**6.** Verfahren nach einem der vorhergehenden Ansprüche, wobei der erste Temperaturzwischenwert der ersten Erhitzungssequenz gleich 400 °C ist.

**7.** Verfahren nach einem der vorhergehenden Ansprüche, wobei der zweite Temperaturzwischenwert der ersten Erhitzungssequenz gleich 550 °C ist.

**8.** Verfahren nach einem der vorhergehenden Ansprüche, wobei die erste Erhitzungssequenz eine zusätzliche isotherme Stufe umfasst, bei der Endtemperatur (TF) der ersten Erhitzungssequenz.

**9.** Verfahren nach einem der vorhergehenden Ansprüche, wobei die vorbestimmte Dauer der isothermen Stufe(n) der ersten Erhitzungssequenz im Bereich von 3 bis 5 Minuten liegt.

**10.** Verfahren nach einem der vorhergehenden Ansprüche, wobei der Temperaturgradient der ersten Erhitzungssequenz im Bereich von 1 bis 50 °C.min$^{-1}$, vorzugsweise von 20 bis 25 °C.min$^{-1}$, liegt.

**11.** Verfahren nach einem der vorhergehenden Ansprüche, wobei der Temperaturgradient der zweiten Erhitzungssequenz im Bereich von 20 bis 40 °C.min$^{-1}$, vorzugsweise von 20 bis 25 °C.min$^{-1}$, liegt.

**Claims**

**1.** Method for characterizing and quantifying the carbon present in a surface formation, starting from a sample representative of said surface formation, **characterized in that** at least the following stages are applied to said sample:

A) said sample is heated according to a first heating sequence under an inert atmosphere, and an amount of hydrocarbon compounds, an amount of CO and an amount of $CO_2$ which are released during said first heating sequence are continuously measured, said first heating sequence under an inert atmosphere comprising at least one isothermal stationary phase of a predetermined duration at an initial temperature (T0), followed by a thermal gradient to reach a final temperature (TF), said initial pyrolysis temperature (T0) being of between 80°C and 200°C, said final pyrolysis temperature (TF) being of between 600°C and 650°C;
B) a residue of said sample resulting from said first heating sequence is heated according to a second heating sequence under an oxidizing atmosphere, and an amount of CO and an amount of $CO_2$ which are released during said second heating sequence are measured, said second heating sequence under an oxidizing atmosphere starting at an initial temperature of between 150°C and 300°C, ending at a final temperature of between 850°C and 1200°C, and following a thermal gradient;
C) a value of a parameter SCmin, representative of a proportion of mineral carbon with respect to the total carbon of said sample, is determined from a ratio of said amount of $CO_2$ released by said residue of said sample beyond an intermediate temperature of said second heating sequence of between 620°C and 680°C to said amount of $CO_2$ released by said residue during said second heating sequence;
D) from said measurements of said amounts of HC, of CO and of $CO_2$ which are released during said first and second heating sequences, parameters are determined which are defined according to formulae of the type:

$$TOC = [S2 * 0.083] + [S3 * \frac{12}{440}] + [(S3CO + \frac{S3'CO}{2}) * \frac{12}{180}] + [S4CO_2 * \frac{12}{440}] + [S4CO * \frac{12}{280}]$$

$$MinC = [S3' * \frac{12}{440}] + [\frac{S3'CO}{2} * \frac{12}{280}] + [S5 * \frac{12}{440}]$$

where S2 represents said amount of HC which are released during said first heating sequence, S3 and S3' represent said amounts of $CO_2$ which are released during said first heating sequence respectively up to and beyond a first intermediate temperature of said first heating sequence of between 370°C and 430°C, *S3CO* and S3'CO represent said amounts of CO which are released during said first heating sequence respectively up to and beyond a second intermediate temperature of said first heating sequence of between 520°C and 580°C, *S4CO2* and *S4CO* represent said amounts respectively of $CO_2$ and of CO which are released during said second heating sequence up to said intermediate temperature of said second heating sequence, and S5 represents said amount of $CO_2$ released during said second heating sequence beyond said intermediate temperature of said second heating sequence.

E) an organic carbon content and/or a mineral carbon content of said sample is/are quantified in the following way:

    i) if said value of said parameter SCmin is less than or equal to a predefined threshold value of said parameter SCmin, said mineral carbon content is zero and said organic carbon content is equal to a sum of said parameters TOC and MinC.

    ii) if said value of said parameter SCmin is greater than said predefined threshold value of said parameter SCmin: said mineral carbon content Cmin is determined according to a formula of the type: *Cmin = MinC - kxTOC;* and/or said organic carbon content Corg is determined according to a formula of the type: *Corg = TOC + kxTOC,* where k is a corrective factor of between 0.04 and 0.12.

2. Method according to Claim 1, in which said predefined threshold value of said parameter SCmin is of between 0.03 and 0.05, and preferably has the value 0.04.

3. Method according to either of the preceding claims, in which said corrective factor k preferentially has the value 0.09.

4. Method according to one of the preceding claims, in which said initial temperature (T0) of said first heating sequence is of between 80°C and 150°C, and preferably has the value 150°C.

5. Method according to one of the preceding claims, in which said initial temperature of said second heating sequence is equal to said initial temperature (T0) of said first heating sequence.

6. Method according to one of the preceding claims, in which said first intermediate temperature of said first heating sequence has the value 400°C.

7. Method according to one of the preceding claims, in which said second intermediate temperature of said first heating sequence has the value 550°C.

8. Method according to one of the preceding claims, in which said first heating sequence comprises an additional isothermal stationary phase, at said final temperature (TF) of said first heating sequence.

9. Method according to one of the preceding claims, in which said predetermined duration of said isothermal stationary phase(s) of said first heating sequence is of between 3 and 5 minutes.

10. Method according to one of the preceding claims, in which said thermal gradient of said first heating sequence is of between 1 and 50°C.min[-1], preferably between 20 and 25°C.min[-1].

11. Method according to one of the preceding claims, in which said thermal gradient of said second heating sequence is of between 20 and 40°C.min[-1], preferably between 20 and 25°C.min[-1].

[Fig. 1A]

[Fig. 1B]

[Fig. 2A]

[Fig. 2B]

[Fig. 2C]

[Fig. 2D]

[Fig. 3A]

[Fig. 3B]

[Fig. 4]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2227797
- US 3953171 A
- FR 2472754
- US 4352673 A

**Littérature non-brevet citée dans la description**

- **BEHAR F.** ; **BEAUMONT V.** ; **DE B.** ; **PENTEADO H.L.** Rock-Eval 6 Technology: Performances and Developments. *Oil & Gas Science and Technology*, 2001, vol. 56, 111-134
- **DISNAR, J.R.** ; **GUILLET, B.** ; **KERAVIS, D.** ; **DI-GIOVANNI, C.** ; **SEBAG, D.** Soil organic matter (SOM) characterization by Rock-Eval pyrolysis: scope and limitations. *Organic Geochemistry*, 2003, vol. 34, 327-343
- **MALOU, O.P.** ; **SEBAG, D.** ; **MOULIN P.** ; **CHEVAL-LIER, T.** ; **BADIANE-NDOUR, N.Y.** ; **THIAM, A.** ; **CHAPUIS-LARDY, L.** The Rock-Eval® signature of soil organic carbon in Arenosols of the Senegalese groundnut Basin. How do agricultural practices matter?. *Agriculture, Ecosystems & Environment*, 2020, vol. 301, 107030, https://doi.org/10.1016/j.agee.2020.107030
- **PILLOT, D.** ; **DEVILLE, E.** ; **PRINZHOFER, A.** Identification and Quantification of Carbonate Species Using Rock-Eval Pyrolysis. *Oil & Gas Science and Technology - Revue d'IFP Energies nouvelles*, 2014, vol. 69, 341-349
- **SEBAG, D.** ; **DISNAR, J.R.** ; **GUILLET, B.** ; **DI GIOVANNI, C.** ; **VERRECCHIA, E.P.** ; **DURAND, A.** Monitoring organic matter dynamics in soil profiles by ''Rock-Eval pyrolysis'': bulk characterization and quantification of degradation. *European Journal of Soil Science*, 2006, vol. 57, 344-355
- **SEBAG, D.** ; **VERRECCHIA, E.P.** ; **CÉCILLON, L.** ; **ADATTE, T.** ; **ALBRECHT, R.** ; **AUBERT, M.** ; **BUREAU, F.** ; **CAILLEAU, G.** ; **COPARD, Y.** ; **DECAËNS, T.** Dynamics of soil organic matter based on new Rock-Eval indices. *Geoderma*, 2016, vol. 284, 185-203
- **MATHIEU** ; **LOZET**. Dictionnaire encyclopédique de Science du Sol. Lavoisier, 2011